# EUROPÄISCHE PATENTSCHRIFT

(11) **EP 1 059 082 B1**
(45) Veröffentlichungstag und Bekanntmachung des Hinweises auf die Patenterteilung: **22.09.2004**
(21) Anmeldenummer: 00110475.1
(22) Anmeldetag: 17.05.2000
(51) Int. Cl.: A61K 7/42, C07C 261/02, C07D 311/04, C08K 5/1545, C08K 5/16

(54) **Verwendung von substituierten Vinyl-tetrahydronaphthalinen und Vinyl-benzotetrahydropyranen als Lichtschutzmittel**
Use of substituted vinyltetrahydronaphthalenes and vinylbenzotetrahydropyrans as sunscreens
Utilisation de vinyltetrahydrohydronaphthalènes et vinylbenzotetrahydropyrannes substituées comme filtres solaires

(30) Priorität: 11.06.1999 DE 19926779
(43) Veröffentlichungstag der Anmeldung: 13.12.2000
(73) Patentinhaber: BASF AKTIENGESELLSCHAFT, 67056 Ludwigshafen (DE)
(72) Erfinder: Prechtl, Frank, Dr., 60318 Frankfurt (DE); Habeck, Thorsten, Dr., 67149 Meckenheim (DE); Westenfelder, Horst, 67435 Neustadt (DE); Wünsch, Thomas, Dr., 67346 Speyer (DE)

(56) Entgegenhaltungen:
- EP-A- 0 303 915
- WO-A-97/15279
- WO-A-99/56740
- DE-A- 19 634 229
- DE-A- 19 755 650
- FR-A- 2 658 075
- US-A- 4 950 467
- US-A- 5 455 265
- US-A- 5 807 900
- FARMER L J ET AL: "Synthesis and structure-activity relationships of potent retinoid X receptor ligands" BIOORGANIC & MEDICINAL CHEMISTRY LETTERS,GB,OXFORD, Bd. 7, Nr. 18, 23. September 1997 (1997-09-23), Seiten 2393-2398, XP004136451 ISSN: 0960-894X

## Beschreibung

Die Erfindung betrifft die Verwendung von vinylsubstituierten Tetrahydronaphthalinen oder Benzotetrahydropyranen, die am Vinylrest elektronenziehende Substituenten tragen als Lichtschutzmittel in kosmetischen Zubereitungen und als Lichtschutzzusätze in Kunststoffen. Die Erfindung betrifft ferner neue lichtschutzwirksame Verbindungen der genannten Art, die als elektronenziehende Substituenten Cyangruppen tragen.

Die in kosmetischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung. In haarkosmetischen Formulierungen soll eine Schädigung der Keratinfaser durch UV-Strahlen vermindert werden.

Das an die Erdoberfläche gelangende Sonnenlicht hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Nun haben dermatologische Untersuchungen gezeigt, daß auch die UV-A-Strahlung durchaus Hautschädigungen und Allergien hervorrufen kann, indem beispielsweise das Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeigt, daß offenbar auch Schädigungen der Erbinformationen in den Zellen durch Sonnenlicht, speziell durch UV-A-Strahlung, hervorgerufen werden. All diese Erkenntnisse lassen daher die Entwicklung effizienter Filtersubstanzen für den UV-A-Bereich notwendig erscheinen.

Es besteht ein wachsender Bedarf an Lichtschutzmitteln für kosmetische und pharmazeutische Zubereitungen, die vor allem als UV-A-Filter dienen können und deren Absorptionsmaxima deshalb im Bereich von ca. 320 bis 380 nm liegen sollten. Um mit einer möglichst geringen Einsatzmenge die gewünschte Wirkung zu erzielen, sollten derartige Lichtschutzmittel zusätzlich eine hohe spezifische Extinktion aufweisen. Außerdem müssen Leichtschutzmittel für kosmetische Präparate noch eine Vielzahl weiterer Anforderungen erfüllen, beispielsweise gute Löslichkeit in kosmetischen Ölen, hohe Stabilität der mit ihnen hergestellten Emulsionen, toxikologische Unbedenklichkeit sowie geringen Eigengeruch und geringe Eigenfärbung.

Eine weitere Anforderung, der Lichtschutzmittel genügen müssen, ist eine ausreichende Photostabilität. Dies ist aber mit den bisher verfügbaren UV-A absorbierenden Lichtschutzmitteln nicht oder nur unzureichend gewährleistet.

In der französischen Patentschrift Nr. 2 440 933 wird das 4-(1,1-Dimethylethyl)-4'-methoxydibenzoylmethan als UV-A-Filter beschrieben. Es wird vorgeschlagen, diesen speziellen UV-A-Filter, der von der Firma GIVAUDAN unter der Bezeichnung "PARSOL 1789" verkauft wird, mit verschiedenen UV-B-Filtern zu kombinieren, um die gesamten UV-Strahlen mit einer Wellenlänge von 280 bis 380 nm zu absorbieren.

Dieser UV-A-Filter ist jedoch, wenn er allein oder in Kombination mit UV-B-Filtern verwendet wird, photochemisch nicht beständig genug, um einen anhaltenden Schutz der Haut während eines längeren Sonnenbades zu gewährleisten, was wiederholte Anwendungen in regelmäßigen und kurzen Abständen erfordert, wenn man einen wirksamen Schutz der Haut gegen die gesamten UV-Strahlen erzielen möchte.

Deshalb sollen gemäß EP-A-0 514 491 die nicht ausreichend photostabilen UV-A-Filter durch den Zusatz von 2-Cyan-3,3-diphenylacrylsäureestern stabilisiert werden, die selbst im UV-B-Bereich als Filter dienen.

Weiterhin wurde gemäß EP-A-0 251 398 schon vorgeschlagen, UV-Aund UV-B-Strahlung absorbierende Chromophore durch ein Bindeglied in einem Molekül zu vereinen. Dies hat den Nachteil, daß einerseits keine freie Kombination von UV-A- und UV-B-Filtern in der kosmetischen Zubereitung mehr möglich ist und daß Schwierigkeiten bei der chemischen Verknüpfung der Chromophore nur bestimmte Kombinationen zulassen.

US 4,950,467 beschreibt die Verwendung von 2,4-Pentadiensäurederivaten als UV-Absorber in kosmetischen Präparaten. Die in dieser Patentschrift bevorzugt genannten Monoaryl-substituierten Verbindungen haben ebenfalls den Nachteil, daß sie nicht genügend photostabil sind.

Es bestand daher die Aufgabe, Lichtschutzmittel für kosmetische Zwecke vorzuschlagen, die vor allem im UV-A-Bereich (und gegebenenfalls alternativ im UV-B-Bereich) mit hoher Extinktion absorbieren, die photostabil sind, eine geringe Eigenfarbe d.h. eine scharfe Bandenstruktur aufweisen und je nach Substituent in Öl oder Wasser löslich sind.

Diese Aufgabe wurde erfindungsgemäß gelöst durch die Verwendung von Verbindungen der Formel I 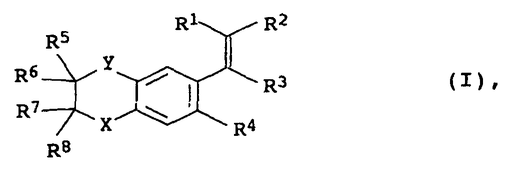 in der
- X: den zweiwertigen Rest des Sauerstoffs, einen Carbonylrest, gegebenenfalls aliphatisch substituiertes Imino oder den Rest -C(R⁹R¹⁰)-
- Y: eine Carbonylgruppe oder den Rest -C(R⁹R¹⁰)-,
- R¹ und R²: gleiche oder verschiedene, Elektronen anziehende Reste, ausgewählt aus der Gruppe bestehend aus Cyan, Alkyl- oder Arylcarbonyl, Alkyloxy- oder Aryloxycarbonyl, gegebenenfalls substituiertem Aminocarbonyl, Alkyl- oder Arylsulfinyl, Alkyl- oder Arylsulfonyl und gegebenenfalls substituiertem Aminosulfonyl,
- R³: ein Wasserstoffatom, eine Cyan-, Hydroxyl-, Carboxyl- oder Aminocarbonylgruppe oder eine gegebenenfalls über eine Sauerstoffbrücke, eine Aminocarbonylbrücke oder Oxycarbonylbrücke gebundenen C₁-C₂₀-Alkylrest oder C₅-C₂₀-Arylrest,
- R⁴: ein Wasserstoffatom, eine Hydroxyl-, eine Aminogruppe oder einen gegebenenfalls über eine Sauerstoffbrücke gebundenen C₁-C₂₀-Alkyl- oder C₅-C₂₀-Arylrest und
- R⁵, R⁶, R⁹ und R¹⁰: unabhängig voneinander Wasserstoff oder C₁-C₂₀-Alkylreste bedeuten
- R⁷ und R⁸: unabhängig voneinander Wasserstoff oder C₁-C₂₀-Alkylreste oder zusammen mit dem Ketten-C-Atom eine Carbonylgruppe bedeuten,
als photostabile UV-Filter in kosmetischen Zubereitungen zum Schutz der menschlichen Haut und menschlicher Haare gegen Sonnenstrahlen, allein oder zusammen mit an sich für kosmetische Zubereitungen bekannten, im UV-Bereich absorbierenden Verbindungen.

Die Alkylreste in den Substituenten R¹ und/oder R² haben in der Regel 1 bis 20 C-Atome und sind vorzugsweise niedermolekulare Alkylreste mit 1 bis 8 C-Atomen und insbesondere Methylreste.

Die Arylreste in den Substituenten R¹ und/oder R² sind isocyclische oder heterocyclische Reste mit 5 bis 10 Ringatomen und 3 bis 20 C-Atomen, wie Phenyl, Naphthyl, Pyridyl, Thiazinyl oder Furanyl, die weiter substituiert sein können.

Alkylreste für R³ und R⁴ und R⁵ bis R¹⁰ sind bevorzugt niedermolekulare Reste mit 1 bis 5 C-Atomen, vorzugsweise Methyl. R³ und/oder R⁴ als Arylrest ist in der Regel ein ein- oder zweikerniger isocyclischer oder heterocyclischer Rest, der noch weiter substituiert sein kann. Beispiele sind Phenyl, Naphthyl, Pyridyl, Thiazinyl oder Furanyl.

Von den Verbindungen der Formel I sind jene bevorzugt, bei denen R³ bis R⁸ Wasserstoff und X und Y -C(R⁹R¹⁰) bedeuten, wobei R⁹ und R¹⁰ für Wasserstoff oder niedermolekulare Alkylreste mit 1 bis 5 C-Atomen stehen bzw.
R³ bis R⁸ Wasserstoff und X Sauerstoff und Y -C(R⁹R¹⁰) bedeuten, wobei R⁹ und R¹⁰ für Wasserstoff oder Alkylreste mit 1 bis 5 C-Atomen stehen.

Besonders bevorzugt sind die Verbindungen der Formel I, in der R¹ und/oder R² die Reste Ethoxycarbonyl-, Acetyl- oder Cyan, R³ Wasserstoff oder Methyl-, R⁴ Wasserstoff oder Methyl-, R⁵ bis R⁸ Wasserstoff, X Sauerstoff oder -C(CH₃)₂- und Y -CH₂- oder -C(CH₃)₂- bedeuten.

Unter den Verbindungen der Formel I sind die Verbindungen der Formel II 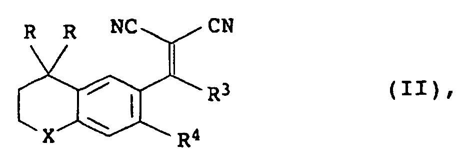 in der
- R³: einen niedermolekularen Alkylrest,
- R⁴: Wasserstoff, einen niedermolekularen Alkylrest oder einen niedermolekularen Alkoxyrest,
- X: den zweiwertigen Rest des Sauerstoffs oder den Rest -C(RR)- bedeuten, wobei R für Wasserstoff oder niedermolekulares Alkyl steht,
neu und werden daher als neue Stoffe beansprucht.

Die Verbindungen der Formel I bzw. II werden in an sich bekannter Weise durch Umsetzung von Verbindungen der Formel III R¹-CH₂-R² mit den substituierten Benzaldehyden der Formel IV 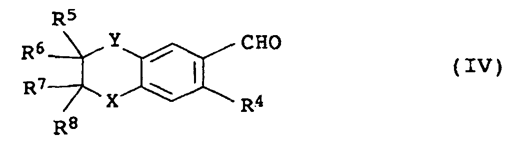 gewonnen, wobei R¹ bis R⁸ die oben angegebenen Bedeutungen haben.

Die Aldehyde der Formel IV sind in der Literatur eingehend beschrieben und können durch Oxidation einer Methylgruppe oder durch Vilsmeyer-Reaktion gewonnen werden.

Die Kondensation zu den bekannten Verbindungen der Formel I bzw. II erfolgt in an sich bekannter Weise durch eine Knoevenagel-Konderisation einer CH-aziden Verbindung wie z.B. Malonsäurediethylester mit dem entsprechenden Naphthylaldehyd und mit katalytischen Mengen von Ammoniumacetat/Eisessig in einem Lösungsmittel wie Methylenchlorid, Cyclohexan, Toluol oder Xylol, welches mit dem entstandenen Reaktionswasser ein abdestillierbares Azeotrop bildet. Die Reaktionstemperaturen richten sich nach dem Siedepunkt des eingesetzten Lösungsmittels und der Reaktivität der Carbonylverbindung. Auf dieselbe Weise können auch die neuen Kondensate mit Malodinitril hergestellt werden; es kann hier teilweise auf die Zugabe eines wasserschleppenden Lösungsmittels verzichtet werden.

Gegenstand der vorliegenden Erfindung sind weiterhin kosmetische Zubereitungen, die 0,1 bis 10 Gew.-%, vorzugsweise 1 bis 7 Gew.-%, bezogen auf die gesamte Menge der kosmetischen Zubereitung, einer oder mehrerer der Verbindungen der Formel I zusammen mit an sich für kosmetische Zubereitungen bekannten, im UV-A- und UV-B-Bereich absorbierenden Verbindungen als Lichtschutzmittel enthalten, wobei die Verbindungen der Formel I in der Regel in geringerer Menge als die UV-B-absorbierenden Verbindungen eingesetzt werden.

Die Lichtschutzmittel enthaltenden kosmetischen Zubereitungen sind in der Regel auf der Basis eines Trägers, der mindestens eine Ölphase enthält. Es sind aber auch Zubereitungen allein auf wäßriger Basis bei Verwendung von Verbindungen mit hydrophilen Substituenten möglich. Demgemäß kommen Öle, Öl-in-Wasser- und Wasser-in-Öl-Emulsionen, Cremes und Pasten, Lippenschutzstiftmassen oder fettfreie Gele in Betracht.

Solche Sonnenschutzpräparate können demgemäß in flüssiger, pastöser oder fester Form vorliegen, beispielsweise als Wasserin-Öl-Cremes, Öl-in-Wasser-Cremes und -Lotionen, Aerosol-Schaumcremes, Gele, Öle, Fettstifte, Puder, Sprays oder alkoholischwäßrige Lotionen.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Übliche kosmetische Hilfsstoffe, die als Zusätze in Betracht kommen können, sind z.B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminiumund/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Der Gesamtanteil der Hilfs- und Zusatzstoffe kann 1 bis 80, vorzugsweise 6 bis 40 Gew.-% und der nicht wäßrige Anteil ("Aktivsubstanz") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

Schließlich können weitere an sich bekannte im UV-Bereich absorbierenden Substanzen mitverwendet werden, sofern sie im Gesamtsystem der erfindungsgemäß zu verwendenden Kombination aus UV-Filtern stabil sind.

Der größte Teil der Lichtschutzmittel in den zum Schutz der menschlichen Epidermis dienenden kosmetischen und pharmazeutischen Zubereitungen besteht aus Verbindungen, die UV-Licht im UV-B-Bereich absorbieren d.h. im Bereich von 280 bis 320 nm. Beispielsweise beträgt der Anteil der erfindungsgemäß zu verwendenden UV-A-Absorber 10 bis 90 Gew.-%, bevorzugt 20 bis 50 Gew.-% bezogen auf die Gesamtmenge von UV-B und UV-A absorbierenden Substanzen.

Als UV-Filtersubstanzen, die in Kombination mit den erfindungsgemäß zu verwendenden Verbindungen der Formel I angewandt werden, kommen beliebige UV-A- und UV-B-Filtersubstanzen in Betracht. Beispielsweise sind zu nennen:

| Nr. | Stoff | CAS-Nr . (=Säure) |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethy-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxyethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfonsäure(Sulisobenzonum) und das Natriumsalz | 4065-45-6 |
| 13 | 3-(4'-Sulfo)benzyliden-bornan-2-on und Salze | 58030-58-6 |
| 14 | 3-Benzylidenbornan-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 2,4,6-Trianilin-(o-carbo-2'-ethylhexyl-1'-oxy)-1,3,5-triazin | 88122-99-0 |
| 18 | 3-Imidazol-4-yl-acrylsäure und ihr Ethylester | 104-98-3 |
| 19 | Menthyl-o-aminobenzoate oder: 5-Methyl-2-(1-methylethyl)-2-aminobenzoate | 134-09-8 |
| 20 | Glyceryl p-aminobenzoat oder: 4-Aminobenzoesäure-1-glyceryl-ester | 136-44-7 |
| 21 | 2,2'-Dihydroxy-4-methoxybenzophenon (Dioxybenzone) | 131-53-3 |
| 22 | 2-Hydroxy-4-methoxy-4-methylbenzophenon (Mexenon) | 1641-17-4 |
| 23 | Triethanolamin Salicylat | 2174-16-5 |
| 24 | Dimethoxyphenylglyoxalsäure oder: 3,4-dimethoxy-phenyl-glyoxal-saures Natrium | 4732-70-1 |
| 25 | 3-(4'Sulfo)benzyliden-bornan-2-on und seine Salze | 56039-58-8 |
| 26 | 2,2 ',4,4'-Tetrahydroxybenzophenon | 131-55-5 |
| 27 | 2,2' -Methylen-bis-[6(2H-benzotriazol-2-yl)-4-(1,1,3,3,-tetramethylbutyl)phenol] | 103597-45-1 |
| 28 | 2,2' -(1,4-Phenylen)-bis-1H-benzimidazol-4,6-disulfonsäure, Na-Salz | 180898-37-7 |
| 29 | 2,4-bis-[4-(2-Ethylhexyloxy)-2-hydroxy]phenyl-6-(4-methoxyphenyl)-(1,3,5)-triazin | 187393-00-6 |
| 30 | 3-(4-Methylbenzyliden)-campher | 36861-47-9 |
| 31 | 4-Bis(polyethoxy)paraaminobenzoesäurepolyethoxyethylester | 113010-52-9 |
| 32 | 2,4-Dihydroxybenzophenon | 131-56-6 |
| 33 | 2,2'-Dihydroxy-4,4'-dimethoxybenzophenon-5,5'-dinatriumsulfonat | 3121-60-6 |

Schließlich sind auch mikronisierte Pigmente wie Titandioxid und Zinkoxid zu nennen.

Zum Schutz menschlicher Haare vor UV-Strahlen können die erfindungsgemäßen Lichtschutzmittel der Formel I in Shampoos, Lotionen, Gelen, Haarsprays, Aerosol-Schaumcremes oder Emulsionen in Konzentrationen von 0,1 bis 10 Gew.-%, bevorzugt 1 bis 7 Gew.-% eingearbeitet werden. Die jeweiligen Formulierungen können dabei u.a. zum Waschen, Färben sowie zum Frisieren der Haare verwendet werden.

Die erfindungsgemäß zu verwendenden Verbindungen zeichnen sich in der Regel durch ein besonders hohes Absorptionsvermögen im Bereich der UV-A-Strahlung mit scharfer Bandenstruktur aus. Weiterhin sind sie gut in kosmetischen Ölen löslich und lassen sich leicht in kosmetische Formulierungen einarbeiten. Die mit den Verbindungen I hergestellten Emulsionen zeichnen sich besonders durch ihre hohe Stabilität, die Verbindungen I selber durch ihre hohe Photostabilität aus, und die mit I hergestellten Zubereitungen durch ihr angenehmes Hautgefühl aus.

Die UV-Filterwirkung der erfindungsgemäßen Verbindungen der Formel I kann auch zur Stabilisierung von Wirk- und Hilfsstoffen in kosmetischen und pharmazeutischen Formulierungen ausgenutzt werden.

Die erfindungsgemäßen Verbindungen eignen sich auch in hervorragender Weise zum Stabilisieren von organischen Materialien gegen die Einwirkung von Licht, Sauerstoff und Wärme.

Als Kunststoffe, die durch die erfindungsgemäßen Verbindungen I stabilisiert werden können, seien beispielsweise genannt:

Polymere von Mono- und Diolefinen, wie z.B. Polyethylen niedriger oder hoher Dichte, Polypropylen, lineares Polybuten-1, Polyisopren, Polybutadien sowie Copolymerisate von Mono- oder Diolefinen oder Mischungen der genannten Polymeren;

Copolymerisate von Mono- oder Diolefinen mit anderen Vinylmonomeren, wie z.B. Ethylen-Alkylacrylat-Copolymere, Ethylen-Alkylmethacrylat-Copolymere, Ethylen-Vinylacetat-Copolymere oder Ethylen-Acrylsäure-Copolymere;

Polystyrol sowie Copolymere von Styrol oder α-Methylstyrol mit Dienen und/oder Acrylderivaten, wie z.B. Styrol-Butadien, Styrol-Acrylnitril (SAN), Styrol-Ethylmethacrylat, Styrol-Butadien-Ethylacrylat, Styrol-Acrylnitril-Methacrylat, Acrylnitril-Butadien-Styrol (ABS) oder Methylmethacrylat-Butadien-Styrol (MBS);

Halogenhaltige Polymere, wie z.B. Polyvinylchlorid, Polyvinylfluorid, Polyvinylidenfluorid sowie deren Copolymere;

Polymere, die sich von α,β-ungesättigten Säuren und deren Derivaten ableiten, wie Polyacrylate, Polymethacrylate, Polyacrylamide und Polyacrylnitrile;

Polymere, die sich von ungesättigten Alkoholen und Aminen bzw. von deren Acrylderivaten oder Acetalen ableiten, z.B. Polyvinylalkohol und Polyvinylacetat;

Polyurethane, Polyamide, Polyharnstoffe, Polyphenylenether, Polyester, Polycarbonate, Polyoxymethylene, Polysulfone, Polyethersulfone und Polyetherketone.

Weiterhin können mit den erfindungsgemäßen Verbindungen I Lacküberzüge stabilisiert werden, z.B. Industrielackierungen. Unter diesen sind Einbrennlackierungen, unter diesen wiederum Fahrzeuglackierungen, vorzugsweise Zweischichtlackierungen, besonders hervorzuheben.

Die erfindungsgemäßen Verbindungen I können in fester oder gelöster Form dem Lack zugesetzt werden. Ihre gute Löslichkeit in Lacksystemen ist dabei von besonderem Vorteil.

Bevorzugt werden die erfindungsgemäßen Verbindungen I zum Stabilisieren von Polyolefinen, insbesondere von Polyethylen, von Polycarbonaten, von Polyamiden, von Polyestern, von Polystyrol, von ABS und von Polyurethanen verwendet. Insbesondere können auch Folien aus den genannten Kunststoffen stabilisiert werden.

Für diese Anwendungsbereiche werden die Verbindungen in Konzentrationen von 0,01 bis 5 Gew.-%, bezogen auf die Menge des Kunststoffs, eingesetzt, bevorzugt in einer Konzentration von 0,02 bis 2 Gew.-%. Die Kombination mit anderen Stabilisatoren, beispielsweise Antioxidantien, Metalldesaktivatoren oder anderen Lichtschutzmitteln sowie mit antistatischen oder flammhemmenden Mitteln, ist oft vorteilhaft. Besonders wichtige Costabilisatoren sind beispielsweise sterisch gehinderte Phenole sowie Phosphite, Phosphonite, Amine und Schwefelverbindungen.

Als geeignete Costabilisatoren kommen z.B. in Betracht:
Phenolische Antioxidationsmittel wie
   2,6-Di-tert.-butyl-4-methylphenol,
   n-Octadecyl-β-(3,5-di-tert.-butyl-4-hydroxyphenyl)-propionat,
   1,1,3-Tris-(2-methyl-4-hydroxy-5-tert.-butylphenyl)-butan,
   1,3, 5-Trimethyl-2,4,6-tris-(3,5-di-tert.-butyl-4-hydroxybenzyl)-benzol,
   1,3,5-Tris-(3,5-di-tert.-butyl-4-hydroxybenzyl)-isocyanurat,
   1,3, 5-Tris-[β-(3,5-di-tert.-butyl-4-hydroxyphenyl)-propionylethyl]-isocyanurat,
   1,3,5-Tris-(2,6-di-methyl-3-hydroxy-4-tert.-butylbenzyl)-isocyanurat und
   Pentaerythrit-tetrakis-[β-(3,5-di-tert.-butyl-4-hydroxy)-propionat],
phosphorhaltige Antioxidantien wie
   Tris-(nonylphenyl)-phosphit, Distearylpentaerythritphosphit,
   Tris-(2,4-di-tert.-butyl-phenyl)-phosphit,
   Tris-(2-tert.-butyl-4-methylphenyl)-phosphit,
   Bis-(2,4-di-tert.-butylphenyl)-pentaerythritdiphosphit und
   Tetrakis-(2,4-di-tert.-butylphenyl)-4,4'-biphenylendiphosphit,
schwefelhaltige Antioxidantien wie
   Dilaurylthiodipropionat,
   Dimyristylthiodipropionat,
   Distearylthiodipropionat,
   Pentaerythrittetrakis-(β-laurylthiopropionat) und
   Pentaerythrittetrakis-(β-hexylthiopropionat),
sterisch gehinderte Amine wie
   Bis-(2,2,6,6-tetramethylpiperidyl)-sebacat,
   Bis-(1,2,2,6,6-pentamethylpiperidyl)-sebacat,
   Bis-(1,2,2,6,6-pentamethylpiperidyl)-ester,
   N,N'-Bis(formyl)-bis(2,2,6,6-tetramethyl-4-piperidyl)-1,6-hexandiamin,
   das Kondensationsprodukt von
   1-Hydroxy-2,2,6,6-tetramethyl-4-hydroxypiperidin und Bernsteinsäure,
   das Kondensationsprodukt von
   N,N'-(2,2,6,6-Tetramethylpiperidyl)-hexamethylendiamin und
   4-tert.-Octylamino-2,6-dichlor,1,3,5-s-triazin,
   Poly-[3-(Eicosyl/Tetracosyl)-1-(2,2,6,6-tetramethylpiperidin-4-yl)-pyrrolidin-2,5-dion],
   Tris-(2,2,6,6-Tetramethylpiperidyl)-nitrilotriacetat,
   Tetrakis-(2,2,6,6-tetramethyl-4-piperidyl)-1,2,3,4-butantetracarbonsäure,
   1,1'-(1,2-Ethandiyl)-bis-(3,3,5,5-tetramethylpiperazinon),
   die Kondensationsprodukte von
   4-Amino-2,2,6,6-tetramethylpiperidinen und Tetramethylolacetylendiharnstoffen sowie
2-(2'-Hydroxyphenyl)-benztriazole,
   2-Hydroxybenzophenone,
   Arylester von Hydroxybenzoesäuren,
   α-Cyanozimtsäurederivate,
   Nickelverbindungen oder
   Oxalsäuredianilide.

Zur Vermischung der erfindungsgemäßen Verbindungen I, vor allem mit Kunststoffen, können alle bekannten Vorrichtungen und Methoden zum Einmischen von Stabilisierungsmitteln oder anderen Zusätzen in Polymere angewandt werden.

### Beispiele

### Beispiel 1

### (Herstellung)

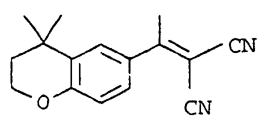

19,2 g (0,094 mol) 6-Acetyl-4,4-dimethyl-3,4-dihydro-2H-1-benzopyran, 6,6 g (0,1 mol) Malodinitril, 1,0 g Ammoniumacetat und 7,0 g Eisessig werden in einem Folben zusammengegeben und 3 h bei 120°C gerührt. Nach dem Abkühlen wird das Produkt durch Säulenchromatographie gereinigt (Kieselgel, Cyclohexan/Essigester 4:1). Man erhielt als Produkt 6,7 g (28 %) eines gelblichen Öls (λₘₐₓ: 346 nm, E¹/₁: 694, Photostabilität 30 min/2 h: 96 %/90 %).

In gleicher Weise sind die Verbindungen der folgenden Tabelle 1 ausgezeichnete Lichtschutzmittel.

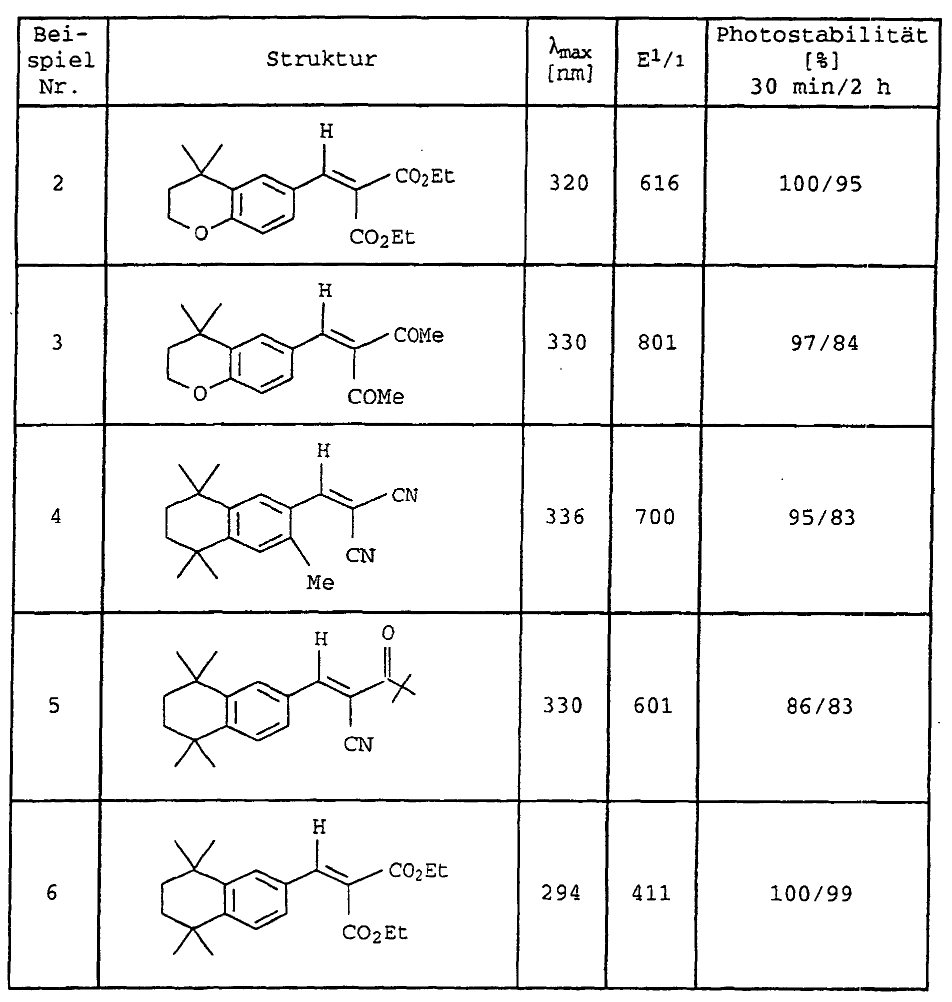

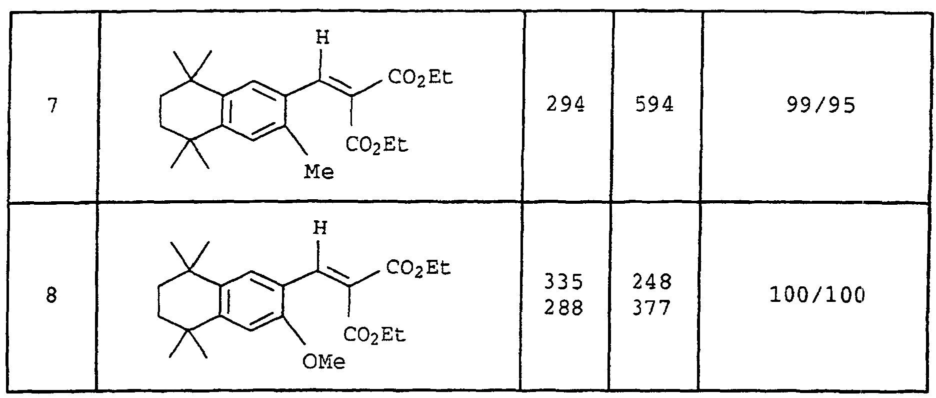

### Beispiel 9

### Standardisierte Methode zur Bestimmung der Photostabilität (Suntest)

Eine 5 gew.-%ige alkoholische Lösung des zu prüfenden Lichtschutzmittels wird mittels einer Eppendorfpipette (20 µl) auf die Auffräsung eines Glasplättchens aufgetragen. Durch die Anwesenheit des Alkohols verteilt sich die Lösung gleichmäßig auf der aufgerauhten Glasoberfläche. Die aufgetragene Menge entspricht der Menge an Lichtschutzmittel, die in Sonnencremes zur Erreichung eines mittleren Lichtschutzfaktors benötigt wird. Bei der Prüfung werden jeweils 4 Glasplättchen bestrahlt. Die Abdampfzeit und die Bestrahlung betragen je 30 Minuten. Die Glasplättchen werden während des Bestrahlens durch eine Wasserkühlung, die sich am Boden des Suntestgerätes befindet, leicht gekühlt. Die Temperatur innerhalb des Suntestgerätes beträgt während der Bestrahlung 40°C. Nachdem die Proben bestrahlt worden sind, werden sie mit Ethanol in einen dunklen 50 ml Meßkolben gewaschen und mit dem Photometer vermessen. Die Blindproben werden ebenso auf Glasplättchen aufgetragen und 30 Minuten bei Raumtemperatur abgedampft. Wie die anderen Proben werden sie mit Ethanol abgewaschen und auf 100 ml verdünnt und vermessen.

### Kosmetische Zubereitungen:

### Beispiel 10

### Allgemeine Herstellvorschrift zur Herstellung von Emulsionen für kosmetische Zwecke

Alle öllöslichen Bestandteile werden in einem Rührkessel auf 85°C erwärmt. Wenn alle Bestandteile geschmolzen sind bzw. als Flüssigphase vorliegen, wird die Wasserphase unter Homogenisieren eingearbeitet. Unter Rühren wird die Emulsion auf ca. 40°C abgekühlt, parfümiert, homogenisiert und dann unter ständigem Rühren auf 25°C abgekühlt.

### Beispiel 11

### (Kosmetische Zubereitung)

| Massengehalt Gew.-% | |
|---|---|
| ad 100 | Wasser |
| 8,00 | Octyl Methoxycinnamat |
| 8,00 | Titanium Dioxid |
| 6,00 | PEG-7-Hydrogenated Castor Öl |
| 5,00 | Verbindung des Beispiels 1 |
| 6,00 | Mineralöl |
| 5,00 | Zink Oxid |
| 5,00 | Isopropyl Palmitat |
| 5,00 | Imidazolidinyl Urea |
| 3,00 | Jojobaöl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 12

### Zusammensetzung für die Lippenpflege

| Massengehalt Gew.-% | |
|---|---|
| ad 100 | Eucerinum anhydricum |
| 10,00 | Glycerin |
| 10,00 | Titanium Dioxid |
| 5,00 | Verbindung des Beispiels 1 |
| 8,00 | Octyl Methoxycinnamat |
| 5,00 | Zink Oxid |
| 4,00 | Castor Öl |
| 4,00 | Pentaerythrithil Stearat/caprat/Caprylat Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 13

### Zusammensetzung für Sunblocker mit Mikropigmenten

| Massengehalt Gew.-% | |
|---|---|
| ad 100 | Wasser |
| 10,00 | Octyl Methoxycinnamat |
| 6,00 | PEG-7-Hydrogenated Castor Öl |
| 6,00 | Titanium Dioxid |
| 5,00 | Verbindung des Beispiels 1 |
| 5,00 | Mineralöl |
| 5,00 | Isoamyl p-Methoxycinnamat |
| 5,00 | Propylen Glykol |
| 3,00 | Jojobaöl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 14

### Fettfreies Gel

| Massengehalt Gew.-% | |
|---|---|
| ad 100 | Wasser |
| 8,00 | Octyl Methoxycinnamat |
| 7,00 | Titaniun Dioxid |
| 5,00 | Verbindung des Beispiels 1 |
| 5,00 | Glycerin |
| 5,00 | PEG-25 PABA |
| 1,00 | 4-Methylbenzyliden Campher |
| 0,40 | Acrylate C₁₀-C₃₀-Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 15

### Sonnencreme (LSF 20)

| Massengehalt Gew.-% | |
|---|---|
| ad 100 | Wasser |
| 8,00 | Octyl Methoxycinnamat |
| 8,00 | Titanium Dioxid |
| 6,00 | PEG-7-Hydrogenated Castor Öl |
| 5,00 | Verbindung des Beispiels 1 |
| 6,00 | Mineralöl |
| 5,00 | Zink Oxid |
| 5,00 | Isopropyl Palmitat |
| 5,00 | Imidazolidinyl Urea |
| 3,00 | Jojobaöl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 16

### Sonnenmilch (LSF 6)

| Massengehalt Gew.-% | |
|---|---|
| ad 100 | Wasser |
| 10,00 | Mineralöl |
| 6,00 | PEG-7-Hydrogenated Castor Öl |
| 5,00 | Isopropyl Palmitat |
| 3,50 | Octyl Methoxycinnamat |
| 5,00 | Verbindung des Beispiels 1 |
| 3,00 | Caprylic/Capric Triglycerid |
| 3,00 | Jojobaöl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 0,30 | Glycerin |
| 0,25 | Methylparaben |
| 0,15 | Propylparaben |
| 0,05 | Tocopherol |

## Patentansprüche

1. Verwendung von Verbindungen der Formel I 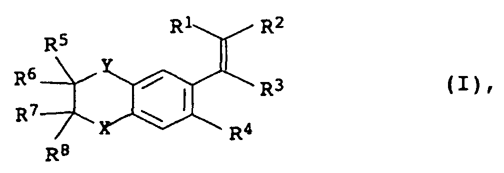 in der
X den zweiwertigen Rest des Sauerstoffs, einen Carbonylrest, gegebenenfalls aliphatisch substituiertes Imino oder den Rest -C(R⁹R¹⁰)-,
Y eine Carbonylgruppe oder den Rest -C(R⁹R¹⁰)-,
R¹ und R² gleiche oder verschiedene, Elektronen anziehende Reste, ausgewählt aus der Gruppe bestehend aus Cyan, Alkyl- oder Arylcarbonyl, Alkyloxy- oder Aryloxycarbonyl, gegebenenfalls substituiertem Aminocarbonyl, Alkyl- oder Arylsulfinyl, Alkyl- oder Arylsulfonyl und gegebenenfalls substituiertem Aminosulfonyl,
R³ ein Wasserstoffatom, eine Cyan-, Hydroxyl-, Carboxyl- oder Aminocarbonylgruppe oder eine gegebenenfalls über eine Sauerstoffbrücke, eine Aminocarbonylbrücke oder Oxycarbonylbrücke gebundenen C₁-C₂₀-Alkylrest oder C₅-C₂₀-Arylrest,
R⁴ ein Wasserstoffatom, eine Hydroxyl-, eine Aminogruppe oder einen gegebenenfalls über.eine Sauerstoffbrücke gebundenen C₁-C₂₀-Alkyl- oder C₅-C₂₀-Arylrest und
R⁵, R⁶, R⁹ und R¹⁰ unabhängig voneinander Wasserstoff oder C₁-C₂₀-Alkylreste bedeuten
R⁷ und R⁸ unabhängig voneinander Wasserstoff oder C₁-C₂₀-Alkylreste oder zusammen mit dem Ketten-C-Atom eine Carbonylgruppe bedeuten,
als photostabile UV-Filter in kosmetischen Zubereitungen zum Schutz der menschlichen Haut und menschlicher Haare gegen Sonnenstrahlen, allein oder zusammen mit an sich für kosmetische Zubereitungen bekannten, im UV-Bereich absorbierenden Verbindungen.

2. Verwendung nach Anspruch 1, **dadurch gekennzeichnet, daß** R³ bis R⁸ Wasserstoff und X und Y -C(R⁹R¹⁰)- bedeuten, wobei R⁹ und R¹⁰ für Wasserstoff oder niedermolekulare Alkylreste mit 1 bis 5 C-Atomen stehen.

3. Verwendung nach Anspruch 1, **dadurch gekennzeichnet, daß** R³ bis R⁸ Wasserstoff und X Sauerstoff und Y -C(R⁹R¹⁰)- bedeuten, wobei R⁹ und R¹⁰ für Wasserstoff oder Alkylreste mit 1 bis 5 C-Atomen stehen.

4. Verwendung nach Anspruch 1, **dadurch gekennzeichnet, daß** R¹ und/oder R² die Reste Ethoxycarbonyl-, Acetyl- oder Cyan, R³ Wasserstoff oder Methyl-, R⁴ Wasserstoff oder Methyl-, R⁵ bis R⁸ Wasserstoff, X Sauerstoff oder -C(CH₃)₂- und Y -CH₂- oder -C(CH₃)₂- bedeuten.

5. Verwendung von Verbindungen der Formel I gemäß Anspruch 1 als Lichtschutzmittel zur Stabilisierung von Kunststoffen.

6. Lichtschutzmittel enthaltende kosmetische Zubereitungen zum Schutz der menschlichen Epidermis oder der menschlichen Haare gegen UV-Licht im Bereich von 280 bis 400 nm, **dadurch gekennzeichnet, daß** sie in einem kosmetisch geeigneten Träger, allein oder zusammen mit an sich für kosmetische Zubereitungen bekannten, im UV-Bereich absorbierenden Verbindungen, Verbindungen der Formel I enthalten, 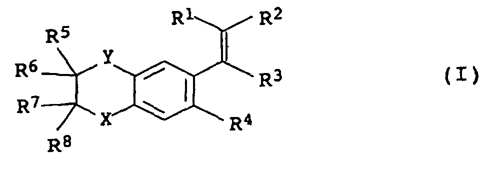 in der
X den zweiwertigen Rest des Sauerstoffs, einen Carbonylrest, gegebenenfalls aliphatisch substituiertes Imino oder den Rest -C(R⁹R¹⁰)-,
Y eine Carbonylgruppe oder den Rest -C(R⁹R¹⁰)-,
R¹ und R² gleiche oder verschiedene, Elektronen anziehende Reste, ausgewählt aus der Gruppe bestehend aus Cyan, Alkyl- oder Arylcarbonyl, Alkyloxy- oder Aryloxycarbonyl, gegebenenfalls substituiertem Aminocarbonyl, Alkyl- oder Arylsulfinyl, Alkyl- oder Arylsulfonyl und gegebenenfalls substituiertem Aminosulfonyl,
R³ ein Wasserstoffatom, eine Cyan-, Hydroxyl-, Carboxyl- oder Aminocarbonylgruppe oder eine gegebenenfalls über eine Sauerstoffbrücke, eine Aminocarbonylbrücke oder Oxycarbonylbrücke gebundenen C₁-C₂₀-Alkylrest oder C₅-C₂₀-Arylrest,
R⁴ ein Wasserstoffatom, eine Hydroxyl-, eine Aminogruppe oder einen gegebenenfalls über eine Sauerstoffbrücke gebundenen C₁-C₂₀-Alkyl- oder C₅-C₂₀-Arylrest und
R⁵, R⁶, R⁹ und R¹⁰ unabhängig voneinander Wasserstoff oder C₁-C₂₀-Alkylreste bedeuten
R⁷ und R⁸ unabhängig voneinander Wasserstoff oder C₁-C₂₀-Alkylreste oder zusammen mit dem Ketten-C-Atom eine Carbonylgruppe bedeuten.

7. Lichtschutzmittel enthaltende kosmetische Zubereitungen nach Anspruch 6, **dadurch gekennzeichnet, daß** sie Verbindungen der Formel I enthalten, in der R³ bis R⁸ Wasserstoff und X und Y -C(R⁹R¹⁰)- bedeuten, wobei R⁹ und R¹⁰ für Wasserstoff oder Alkylreste mit 1 bis 5 C-Atomen stehen.

8. Lichtschutzmittel enthaltende kosmetische Zubereitungennach Anspruch 6, **dadurch gekennzeichnet, daß** sie Verbindungen der Formel I enthalten, in der R³ bis R⁸ Wasserstoff und X Sauerstoff und Y -C(R⁹R¹⁰)- bedeuten, wobei R⁹ und R¹⁰ für Wasserstoff oder Alkylreste mit 1 bis 5 C-Atomen stehen.

9. Lichtschutzmittel enthaltende kosmetische Zubereitungen nach Anspruch 6, **dadurch gekennzeichnet, daß** sie Verbindungen der Formel I enthalten, in der R¹ und/oder R² die Reste Ethoxycarbonyl-, Acetyl- oder Cyan, R³ Wasserstoff oder Methyl-, R⁴ Wasserstoff oder Methyl-, R⁵ bis R⁸ Wasserstoff, X Sauerstoff oder -C(CH₃)₂- und Y -CH₂- oder -C(CH₃)₂- bedeuten.

10. Neue als Lichtschutzmittel geeignete Verbindungen der Formel II 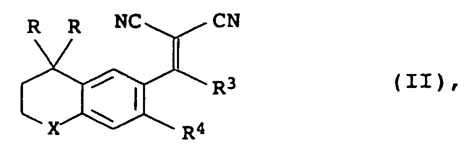 in der
R³ einen niedermolekularen Alkylrest mit 1 bis 5 C-Atomen,
R⁴ Wasserstoff, einen niedermolekularen Alkylrest mit 1 bis 5 C-Atomen oder einen niedermolekularen Alkoxyrest mit 1 bis 5 C-Atomen,
X den zweiwertigen Rest des Sauerstoffs oder den Rest - C(RR)- bedeuten, wobei R Wasserstoff oder niedermolekulares Alkyl mit 1 bis 5 C-Atomen bedeutet.

## Claims

1. The use of compounds of the formula I 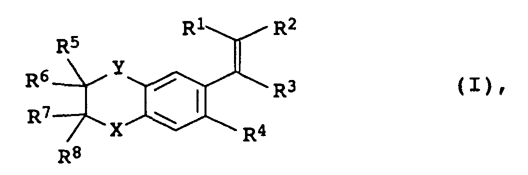 in which
X is the divalent radical of oxygen, a carbonyl radical, optionally aliphatically substituted imino or the radical -C(R⁹R¹⁰)-,
Y is a carbonyl group or the radical -C(R⁹R¹⁰)-,
R¹ and R² are identical or different electron-withdrawing radicals, chosen from the group consisting of cyano, alkyl- or arylcarbonyl, alkyloxy- or aryloxycarbonyl, optionally substituted aminocarbonyl, alkyl- or arylsulfinyl, alkyl- or arylsulfonyl and optionally substituted aminosulfonyl,
R³ is a hydrogen atom, a cyano, hydroxyl, carboxyl or aminocarbonyl group or a C₅-C₂₀-aryl radical or C₁-C₂₀-alkyl radical optionally bonded via an oxygen bridge, an aminocarbonyl bridge or oxycarbonyl bridge,
R⁴ is a hydrogen atom, a hydroxyl group, an amino group, or a C₅-C₂₀-aryl radical or C₁-C₂₀-alkyl radical optionally bonded via an oxygen bridge, and
R⁵, R⁶, R⁹ and R¹⁰ independently of one another are hydrogen or C₁-C₂₀-alkyl radicals,
R⁷ and R⁸ independently of one another are hydrogen or C₁-C₂₀-alkyl radicals or together with the chain carbon atom are a carbonyl group,
as photostable UV filters in cosmetic preparations for protecting human skin and human hair from solar rays, alone or together with compounds which absorb in the UV region and are known per se for cosmetic preparations.

2. The use as claimed in claim 1, wherein R³ to R⁸ are hydrogen, and X and Y are -(CR⁹R¹⁰)-, where R⁹ and R¹⁰ are hydrogen or low molecular weight alkyl radicals having from 1 to 5 carbon atoms.

3. The use as claimed in claim 1, wherein R³ to R⁸ are hydrogen, and X is oxygen and Y is -C(R⁹R¹⁰)-, where R⁹ and R¹⁰ are hydrogen or alkyl radicals having from 1 to 5 carbon atoms.

4. The use as claimed in claim 1, wherein R¹ and/or R² are the radicals ethoxycarbonyl, acetyl or cyano, R³ is hydrogen or methyl, R⁴ is hydrogen or methyl, R⁵ to R⁸ are hydrogen, X is oxygen or -C(CH₃)₂- and Y is -CH₂- or -C(CH₃)₂-.

5. The use of compounds of the formula I as claimed in claim 1 as light protection agents for stabilizing plastics.

6. A cosmetic preparation comprising light protection agents, for protecting the human epidermis or human hair from UV light in the range from 280 to 400 nm, which comprises, in a cosmetically suitable carrier, alone or together with compounds which absorb in the UV region and are known per se for cosmetic preparations, compounds of the formula I 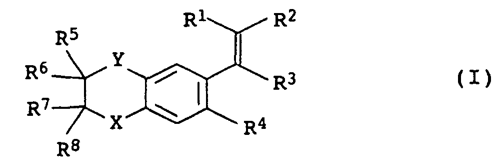 in which
X is the divalent radical of oxygen, a carbonyl radical, optionally aliphatically substituted imino or the radical -C(R⁹R¹⁰)-,
Y is a carbonyl group or the radical -C(R⁹R¹⁰)-,
R¹ and R² are identical or different electron-withdrawing radicals, chosen from the group consisting of cyano, alkyl- or arylcarbonyl, alkyloxy- or aryloxycarbonyl, optionally substituted aminocarbonyl, alkyl- or arylsulfinyl, alkyl- or arylsulfonyl and optionally substituted aminosulfonyl,
R³ is a hydrogen atom, a cyano, hydroxyl, carboxyl or aminocarbonyl group or a C₅-C₂₀-aryl radical or C₁-C₂₀-alkyl radical optionally bonded via an oxygen bridge, an aminocarbonyl bridge or oxycarbonyl bridge,
R⁴ is a hydrogen atom, a hydroxyl group, an amino group, or a C₅-C₂₀-aryl radical or C₁-C₂₀-alkyl radical optionally bonded via an oxygen bridge, and
R⁵, R⁶, R⁹ and R¹⁰ independently of one another are hydrogen or C₁-C₂₀-alkyl radicals,
R⁷ and R⁸ independently of one another are hydrogen or C₁-C₂₀-alkyl radicals or together with the chain carbon atom are a carbonyl group.

7. A cosmetic preparation comprising light protection agents as claimed in claim 6, which comprises compounds of the formula I in which R³ to R⁸ are hydrogen and X and Y are -C(R⁹R¹⁰)-, where R⁹ and R¹⁰ are hydrogen or alkyl radicals having from 1 to 5 carbon atoms.

8. A cosmetic preparation comprising light protection agents as claimed in claim 6, which comprises compounds of the formula I in which R³ to R⁸ are hydrogen and X is oxygen and Y is -C(R⁹R¹⁰)-, where R⁹ and R¹⁰ are hydrogen or alkyl radicals having from 1 to 5 carbon atoms.

9. A cosmetic preparation comprising light protection agents as claimed in claim 6, which comprises compounds of the formula I in which R¹ and/or R² are the radicals ethoxycarbonyl, acetyl or cyano, R³ is hydrogen or methyl, R⁴ is hydrogen or methyl, R⁵ to R⁸ are hydrogen, X is oxygen or -C(CH₃)₂- and Y is -CH₂- or -C(CH₃)₂-.

10. A novel compound, suitable as light protection agent, of the formula II 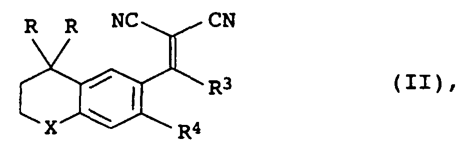 in which
R³ is a low molecular weight alkyl radical having from 1 to 5 carbon atoms,
R⁴ is hydrogen, a low molecular weight alkyl radical having from 1 to 5 carbon atoms or a low molecular weight alkoxy radical having from 1 to 5 carbon atoms,
X is the divalent radical of oxygen or the radical -C(RR)-, where R is hydrogen or low molecular weight alkyl having from 1 to 5 carbon atoms.

## Revendications

1. Utilisation de composés de formule I 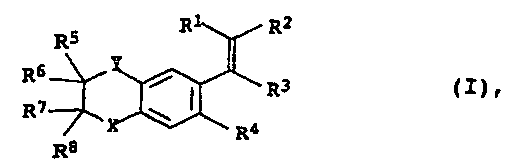 dans laquelle
X désigne le reste divalent de l'oxygène, un reste carbonyle, un reste imino à substitution aliphatique éventuelle ou le reste -C(R⁹R¹⁰)-,
Y représente un groupe carbonyle ou le reste -C(R⁹R¹⁰)-,
R¹ et R² identiques ou différents, désignent des restes électro-attracteurs, choisis dans le groupe consistant en cyano, alkyl- ou arylcarbonyle, alkyloxy- ou aryloxycarbonyle, aminocarbonyle éventuellement substitué, alkyl- ou arylsulfinyle, alkyl- ou arylsulfonyle, et aminosulfonyle éventuellement substitué,
R³ désigne un atome d'hydrogène, un groupe cyano, hydroxy, carboxyle ou aminocarbonyle, ou un reste alkyle en C₁-C₂₀ ou un reste aryle en C₅-C₂₀, éventuellement lié par un pont oxygène, par un pont aminocarbonyle ou par un pont oxycarbonyle,
R⁴ désigne un atome d'hydrogène, un groupe hydroxyle, un groupe amino ou un reste alkyle en C₁-C₂₀ ou aryle en C₅-C₂₀, éventuellement lié par un pont oxygène, et
R⁵, R⁶, R⁹ et R¹⁰ représentent, indépendamment l'un de l'autre, l'hydrogène ou des restes alkyle en C₁-C₂₀,
R⁷ et R⁸ représentent, indépendamment l'un de l'autre, l'hydrogène ou des restes alkyle en C₁-C₂₀, ou désignent un groupe carbonyle conjointement avec l'atome de carbone de la chaîne,
comme filtre UV photostable dans des préparations cosmétiques pour la protection de la peau humaine et de la chevelure humaine contre les rayons solaires, seuls ou conjointement avec des composés absorbant dans le domaine UV, connus en soi pour les préparations cosmétiques.

2. Utilisation selon la revendication 1, **caractérisée par le fait que** R³ à R⁸ désignent l'hydrogène, et X et Y représentent -C(R⁹R¹⁰)-, tandis que R⁹ et R¹⁰ désignent l'hydrogène ou des restes alkyle de faible masse moléculaire ayant 1 à 5 atomes de carbone.

3. Utilisation selon la revendication 1, **caractérisée par le fait que** R³ à R⁸ désignent l'hydrogène, et X est l'oxygène et Y représente -C(R⁹R¹⁰)-, tandis que R⁹ et R¹⁰ désignent l'hydrogène ou des restes alkyle ayant 1 à 5 atomes de carbone.

4. Utilisation selon la revendication 1, **caractérisée par le fait que** R¹ et/ ou R² représentent les restes éthoxycarbonyle, acétyle ou cyano, R³ désigne l'hydrogène ou le méthyle, R⁴ représente l'hydrogène ou le méthyle, R⁵ à R⁸ désignent l'hydrogène, X désigne l'oxygène ou -C(CH₃)₂-, et Y représente -CH- ou -C(CH₃)₂-.

5. Utilisation de composés de formule I selon la revendication 1 comme agents de protection contre la lumière pour la stabilisation de matières plastiques.

6. Préparations cosmétiques contenant des agents de protection contre la lumière, pour la protection de l'épiderme humain ou de la chevelure humaine contre la lumière UV dans le domaine de 280 à 400 nm, **caractérisées par le fait qu'**elles contiennent dans un support approprié en cosmétique, seuls ou en combinaison avec des composés absorbant dans le domaine UV, connus en soi pour des préparations cosmétiques, des composés de formule I 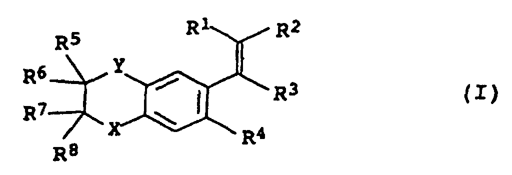 dans laquelle
X désigne le reste divalent de l'oxygène, un reste carbonyle, un reste imino à substitution aliphatique éventuelle ou le reste -C(R⁹R¹⁰)-,
Y représente un groupe carbonyle ou le reste -C(R⁹R²⁰)-,
R¹ et R² identiques ou différents, désignent des restes électro-attracteurs, choisis dans le groupe consistant en cyano, alkyl- ou arylcarbonyle, alkyloxy- ou aryloxycarbonyle, aminocarbonyle éventuellement substitué, alkyl- ou arylsulfinyle, alkyl- ou arylsulfonyle, et aminosulfonyle éventuellement substitué,
R³ désigne un atome d'hydrogène, un groupe cyano, hydroxy, carboxyle ou aminocarbonyle, ou un reste alkyle en C₁-C₂₀ ou un reste aryle en C₅-C₂₀, éventuellement lié par un pont oxygène, par un pont aminocarbonyle ou par un pont oxycarbonyle,
R⁴ désigne un atome d'hydrogène, un groupe hydroxyle, un groupe amino ou un reste alkyle en C₁-C₂₀ ou aryle en C₅-C₂₀, éventuellement lié par un pont oxygène, et
R⁵, R⁶, R⁹ et R¹⁰ représentent, indépendamment l'un de l'autre, l'hydrogène ou des restes alkyle en C₁-C₂₀,
R⁷et R⁸ représentent, indépendamment l'un de l'autre, l'hydrogène ou des restes alkyle en C₁-C₂₀, ou désignent un groupe carbonyle conjointement avec l'atome de carbone de la chaîne.

7. Préparations cosmétiques contenant des agents de protection contre la lumière selon la revendication 6, **caractérisées par le fait qu'**elles contiennent des composés de formule I, dans laquelle R³ à R⁸ désigne l'hydrogène, et X et Y représentent -C(R⁹R¹⁰)-, tandis que R⁹ et R¹⁰ désignent l'hydrogène ou des restes alkyle ayant 1 à 5 atomes de carbone.

8. Préparations cosmétiques contenant des agents de protection contre la lumière selon la revendication 6, **caractérisées par le fait qu'**elles contiennent des composés de formule I, dans laquelle R³ à R⁸ désigne l'hydrogène, et X désigne l'oxygène et Y représente -C(R⁹R¹⁰)-, tandis que R⁹ et R¹⁰ désignent l'hydrogène ou des restes alkyle ayant 1 à 5 atomes de carbone.

9. Préparations cosmétiques contenant des agents de protection contre la lumière selon la revendication 6, **caractérisées par le fait qu'**elles contiennent des composés de formule I, dans laquelle R¹ et/ou R² désignent les restes éthoxy, carbonyle acétyle ou cyano, R³ représente l'hydrogène ou le méthyle, R⁴ représente l'hydrogène ou le méthyle, R⁵ à R⁸ désignent l'hydrogène, X représente l'oxygène ou -C(CH₃)₂-, et Y désigne -CH₂- ou -C(CH₃)₂-.

10. Nouveaux composés convenant comme agents de protection contre la lumière de formule II 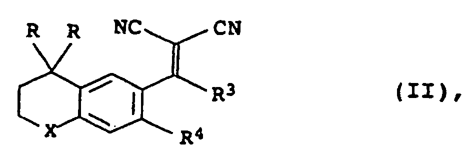 dans laquelle
R³ désigne un reste alkyle de faible masse moléculaire ayant 1 à 5 atomes de carbone,
R⁴ représente l'hydrogène, un reste alkyle de faible masse moléculaire ayant 1 à 5 atomes de carbone, ou un reste alcoxy de faible masse moléculaire ayant 1 à 5 atomes de carbone,
X désigne le reste divalent de l'oxygène ou le reste -C(RR)-, tandis que R représente l'hydrogène ou un alkyle de faible masse moléculaire ayant 1 à 5 atomes de carbone.
